# EUROPÄISCHE PATENTSCHRIFT

(11) **EP 1 430 840 B1**
(45) Veröffentlichungstag und Bekanntmachung des Hinweises auf die Patenterteilung: **29.09.2010**
(21) Anmeldenummer: 03024835.5
(22) Anmeldetag: 31.10.2003
(51) Int. Cl.: A61B 17/08, A61B 17/10, A61B 17/04

(54) **Medizinischer Clip**
Surgical clip
Pince chirurgicale

(30) Priorität: 19.12.2002 DE 10259411
(43) Veröffentlichungstag der Anmeldung: 23.06.2004
(73) Patentinhaber: Karlsruher Institut für Technologie, 76131 Karlsruhe (DE)
(72) Erfinder: Kissel, Christian, 76356 Weingarten (DE); Fischer, Harald, Dr., 76356 Weingarten (DE)

(56) Entgegenhaltungen:
- EP-A- 0 559 417
- EP-A- 0 679 368
- WO-A-00/56227
- US-A- 4 226 239
- US-A- 4 791 707
- US-A- 5 779 720
- US-A1- 2002 055 750
- US-A1- 2002 082 641

## Beschreibung

Die Erfindung betrifft einen medizinischen Clip gemäß des Anspruchs 1 sowie eine Vorrichtung zum Applizieren eines medizinischen Clips gemäß des Anspruchs 6.

Im Bereich der Chirurgie sind eine Vielzahl von medizinischen Clips sowie Vorrichtung zum applizieren derartiger Clips bekannt. Sie dienen insbesondere zum Abklemmen von Gefäßen sowie zum Zusammenhalten von Wunden, aber auch zum fixieren von unterschiedlichsten Implantaten im menschlichen Körpern. Gegenüber anderen für diesen Zweck vorhandenen chirurgischen Verfahren, insbesondere den Nähverfahren liegt der besondere Vorteil eines Clipeinsatzes im vergleichsweise geringen Zeitaufwand für eine Applikation. Dies ist beispielweise bei einem Verschließen einer starken Blutung von entscheidender Bedeutung für den Erfolg eines Eingriffs, wobei alle Nachteile von Clipverfahren, wie z.B. eine höhere lokale Belastung des zu klammernden Gewebes oder eine zusätzliche Verletzungsgefahr durch den applizierten Clip, in den Hintergrund treten.

Grundsätzlich unterscheidet man elastisch und plastisch verformbare, ein- und mehrteilige sowie arretierbare und nicht arretierbare medizinische Clips.

Die vorliegende Erfindung betrifft einen einteiligen, elastisch verformbaren Clip ohne Arretierung. Ein derartiger Clip weist prinzipielle Merkmale einer Klammer auf, d.h. sie besteht im Wesentlichen aus zwei gegeneinander vorgespannten und wirkenden Greifelementen, welche für eine Applikation am Patienten elastisch aufzuspannen sind. Der besondere Vorteil dieser Bauform liegt darin, dass ein derartiger Clip reversibel einsetzbar ist, d.h. nach einer Applikation durch ein einfaches erneutes elastisches Aufbiegen wieder entfernbar ist.

In der DE 41 10 123 A1 ist eine elastische Klammer zum Zusammenhalten von Wunden sowie einer Vorrichtung zum Applizieren dieser Klammer offenbart. Die Klammer weist im Wesentlichen die Form einer drahtförmigen Krampe auf und wird bei der Applikation elastisch aufgebogen mit seinen beiden Enden beidseitig der zu verschließenden Wunde in das umliegende Gewebe eingedrückt. Nach der Applikation werden die beiden Wundränder durch die elastische Kraft der Klammer aneinandergedrückt, wobei eine zusätzliche Verzahnung der Klammerarme als Widerhaken ein mögliches Herausrutschen der Klammer aus dem Gewebe zusätzlich erschwert. Die elastische Klammer ist somit für eine dauerhafte Klammerung ausgelegt und eignet sich für den zuvor beschriebenen reversiblen Einsatz nur eingeschränkt einsetzbar.

Die WO-A-00/56227 beschreibt eine Klemmvorrichtung mit sich gegenüberliegenden Stirnseiten in der Form von Spikes und einem zentralen Biegeelement.

Die US-A-2002/0055750 beschreibt einen Clip in der Form eines gekrümmten Plättchens. Die sich gegenüberstehenden Greifelemente liegen in der Krümmungsebene.

Die Aufgabe der vorliegenden Erfindung liegt davon ausgehend darin, einen einteiligen, elastisch verformbaren medizinischen Clip ohne Arretierung vorzuschlagen, welcher die genannten Einschränkungen hinsichtlich eines reversiblen Einsatzes nicht aufweist.

Gelöst wird diese Aufgabe durch die Merkmale des ersten Patentanspruchs. Die Unteransprüche beschreiben vorteilhafte Ausgestaltungen der Erfindung.

Erfindungsgemäß wird ein Clip mit zwei gegeneinander vorgespannten Greifelementen vorgeschlagen, welcher bei einer Applikation das Gewebe lediglich einklemmt und zuverlässig fixiert, ohne dabei in das Gewebe eingedrückt werden zu müssen. Ferner ist dieser Clip auf einen Faden oder anderen Gegenständen aufklemmbar. Vorzugsweise ist der Clip aus einem Plättchen aus einer Formgedächtnislegierung beispielsweise mit einem Erodier- oder Laserverfahren herausgearbeitet. Grundsätzlich der Clip mit einer Zange wieder entfernbar.

Die Erfindung wird anhand der folgenden Ausführungsformen mit Hilfe der folgenden Figuren erläutert. Es zeigen
Fig. 1 ein Plättchen mit einer H-förmigen Struktur als Durchbruch,
Fig. 2a bis c eine erste Ausführungsform eines Clips in entspanntem und gespanntem Zustand sowie diese beispielhaft appliziert an einen Faden,
Fig. 3a bis c eine zweite Ausführungsform eines Clips in entspanntem und gespanntem Zustand sowie diese beispielhaft appliziert als Wundverschluss,
Fig. 4a und b Schnittdarstellungen je einer Vorrichtung mit Magazin zum Setzen der Clips, sowie
Fig. 5a und b eine Ausführungsform gemäß Fig. 4 a mit einer Zusatzeinrichtung zum Applizieren sowie eine Applikation eines Clips und eines Fadens mit T-Anker.

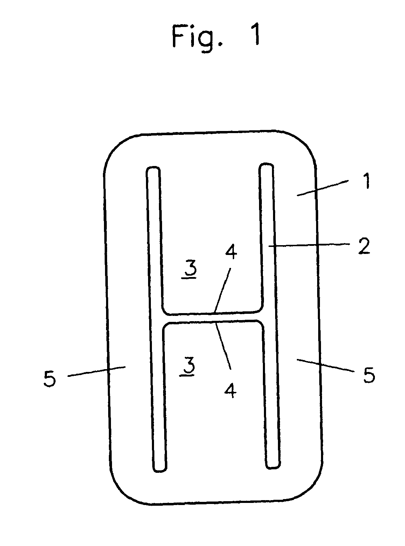

Der Clip besteht, wiedergegeben in den Fig. 1 bis 3, aus einem einzigen Bauteil, nämlich einem Plättchen 1 aus einem elastischen biokompatiblen Material, welches eine Krümmung sowie einen Durchbruch 2, in den Figuren in der Gestalt eines H, unter Bildung zweier Laschen 3 aufweist. Die beiden Laschen sind abgebogen und bilden mit ihren jeweils gegenüber liegenden Stirnseiten 4 die Greifelemente des Clips. Die Bereiche das Plättchens, welche jeweils seitlich außerhalb der beiden parallel zueinander angeordneten Linien der dargestellten H-förmigen Struktur angeordnet sind und die beiden Laschen auf beiden Seiten miteinander verbinden, bilden Biegeelemente 5, welche die elastische Vorspannung auf die Greifelemente des Clips gegeneinander bewirken. Die Krümmung ist vorzugsweise C- oder U-förmig, wobei grundsätzlich alle Krümmungswinkel zwischen 0 und 180° eingeschlossen sind. Es empfiehlt sich, alle Ecken des Clips, zumindest aber die außenliegenden Ecken des Plättchen 1 zur Minimierung einer Verletzungsgefahr durch den Clip abzurunden.

Der wesentlicher Vorteil der Erfindung liegt in seinem einfachen Aufbau, welcher einer wirtschaftlichen Fertigung in großen Stückzahlen sehr entgegenkommt. Das Plättchen 1 als Grundkörper des Clips ist technisch einfach und wirtschaftlich in großer Zahl aus Blechen herstellbar. Ferner kann der Durchbruch 2 durch einen einfachen Stanzvorgang ggf. zusammen mit einem Abbiegen der beiden Laschen 3 eingeprägt werden, wobei hierfür ein Temperaturbereich zu wählen ist, bei dem das biokompatible Material möglichst ein duktiles Dehnungsverhalten und eine geringe Festigkeit aufweist. Im Anschluss erhält der Clip je nach Material thermomechanisch oder auf anderem Wege seine endgültige Gestalt.

Zum Herausschneiden der Plättchen 1 sowie des Durchbruchs 2 eignen sich besonders auch Erosions- oder Laserbearbeitungsverfahren, was einer Miniaturisierung der Clips sehr entgegenkommt.

Diese Gestaltung bewirkt in vorteilhafter Weise, dass zwei Wundränder oder andere Gegenstände zwischen den Stirnseiten 4 der Laschen 3, d.h. in die beiden Greifelemente einspannbar sind und allein durch eine hierdurch erzeugte Druckspannung aneinandergepresst werden. Insbesondere ermöglichen eine hohen Steifigkeit des Plättchens 1 kombiniert mit einer geringen Abmessung des Clips eine zuverlässige Klammerung mit hohen Kräften, wobei diese allein durch eine einfach umzusetzende Auslegung der Clips konstruktiv genau und reproduzierbar einstellbar sind. Ein Hineindrücken mindestens eines Greifelementes in Gewebebereiche ist wie auch ein Einsatz von Widerhaken gegen ein Herausrutschen der Greifelemente aus dem Gewebe nicht mehr erforderlich.

Die Klemmwirkung des Clips kann dadurch verbessert werden, indem die Stirnseiten 4 der Laschen 3 eine Verzahnung, einen rutschfesten Belag, eine raue Oberfläche oder eine Riffelung erhalten. Herstellungstechnisch muss im Fall einer Verzahnung der Querstrich der H-förmigen Struktur gezackt gestaltet sein.

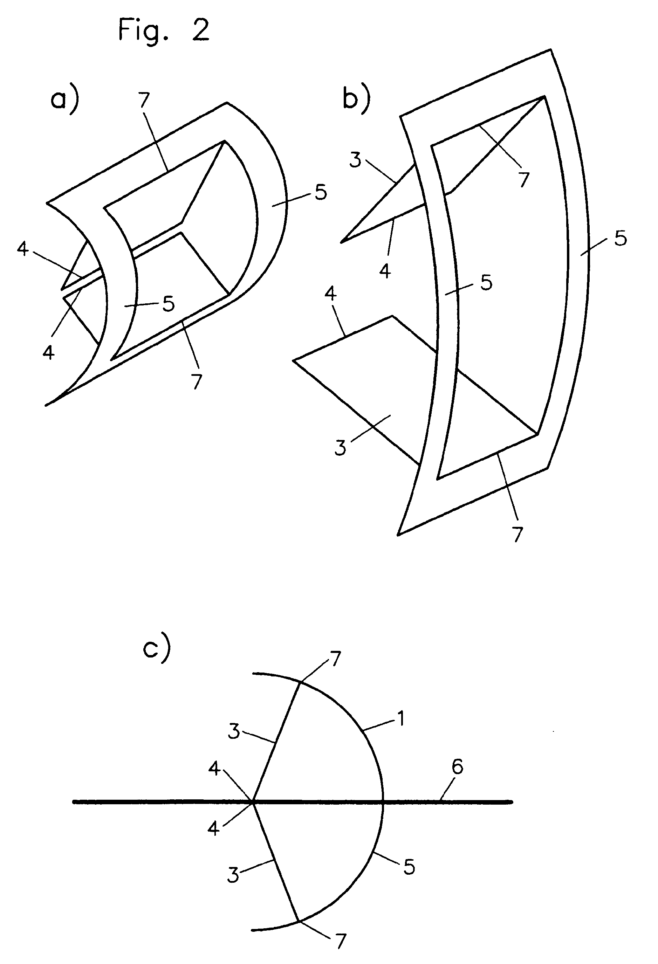

Fig. 2a bis c zeigen eine erste Ausführungsform des Clips, bei der die Laschen 3 in Innenrichtung der Krümmung (C-Form) eingebogen sind. Fig. 2a zeigt den entlasteten, d. h. geschlossenen Clip, welcher eine ausgeprägten Krümmungswinkel aufweist und wobei sich die Stirnseiten 4 der Laschen 3 berühren oder zumindest nahe kommen. Zum Applizieren müssen die Clips gegen die vorgegebene Krümmung elastisch aufgebogen werden, wobei sich die Stirnseiten 4 auseinanderbewegen (vgl. Fig. 2b). Als Applikationsvorrichtung eignet sich insbesondere eine nicht in den Figuren dargestellte Doppelzange, welche mit jeweils zwei Zangengreifern beide Biegelemente 5 synchron einspannt und elastisch aufweitet.

Fig. 2c gibt mit der Fixierung eines Fadens 6 eine bevorzugte Anwendung für den Clip der ersten Ausführungsform wieder. Die beiden Laschen stehen dabei nicht fluchtend zueinander und sind im Clip an den Knickstellen 7, welche nicht mit den Stirnseiten 4 auf einer Geraden liegen, aufgehängt. In Folge dessen werden die Stirnseiten bei einem Durchziehen des eingespannten Fadens in einer Richtung (in Fig. 2c nach links) weiter auseinandergedrückt, während sie sich bei einem Zug des eingespannten Fadens in die Gegenrichtung (in Fig. 2c nach rechts) fester in den Faden eindrücken, dort verkanten und somit ein Hindurchziehen des Fadens verhindern. Somit fixiert der Clip nicht nur zuverlässig, sondern lässt noch ein zusätzliche Nachschiebemöglichkeit des Clips auf dem Faden zu. Ferner dienen die beiden Biegelemente 5 als Widerlager, welche sich beispielsweise bei der Sicherung eines Fadenendes durch den Clip bei einer Vernähung von Wundrändern den Clip gegen das umgebende Gewebe abstützen.

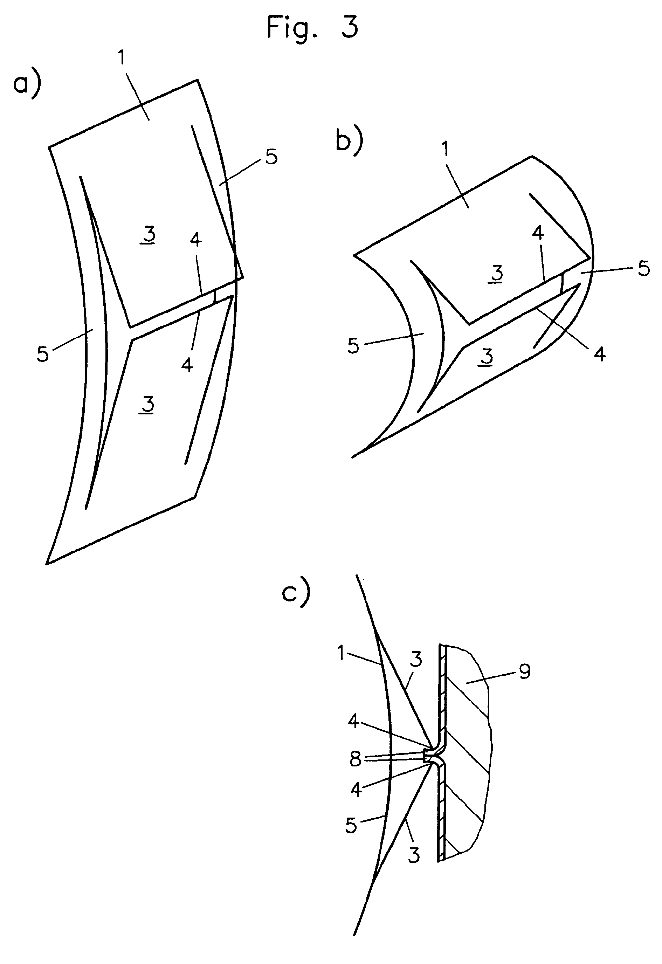

Eine zweite Ausführungsform des Clips zeigen die Figuren 3a bis c. Im Gegensatz zu der ersten Ausführungsform sind die Laschen 3 nicht in den gekrümmten Clip, sondern nach außen abgebogen. Die Laschen stehen entweder, wie in den Figuren 3a bis c dargestellt, tangential am gekrümmten Clip ab oder weisen wie die ersten Ausführungsform definierte Knickstellen auf. Fig. 3a zeigt den entlasteten, d. h. geschlossenen Clip, welcher einen vergleichsweise geringen Krümmungswinkel aufweist und wobei sich die Stirnseiten 4 der Laschen 3 nahe kommen. Zum Applizieren müssen die Clips weiter elastisch gekrümmt werden, wobei sich die Stirnseiten 4 auseinanderbewegen (vgl. Fig. 3b). Als Applikationsvorrichtung eignen sich Zangen gewöhnlicher Bauform, welche den Clip an seinen Enden zusammendrückt.

Fig. 3c zeigt den Clip der zweiten Ausführungsform in der Anwendung als Wundverschluss. Die Wundränder 8 eines Organs 9 eines Patienten werden dabei durch die Stirnseiten 4 des Clips zusammengedrückt, wobei der besondere Vorteil dieser Ausführungsform aufgrund günstiger Hebelverhältnisse in seiner hohen Klemmkraft liegt.

Bei einer Ausgestaltung der Stirnseiten 4 als Schneiden eignet sich vor allem die zweite Ausführungsform des Clips auch als miniaturisiertes Schneidgerät, beispielsweise für ein Durchtrennen von Gewebe in der Chirurgie.

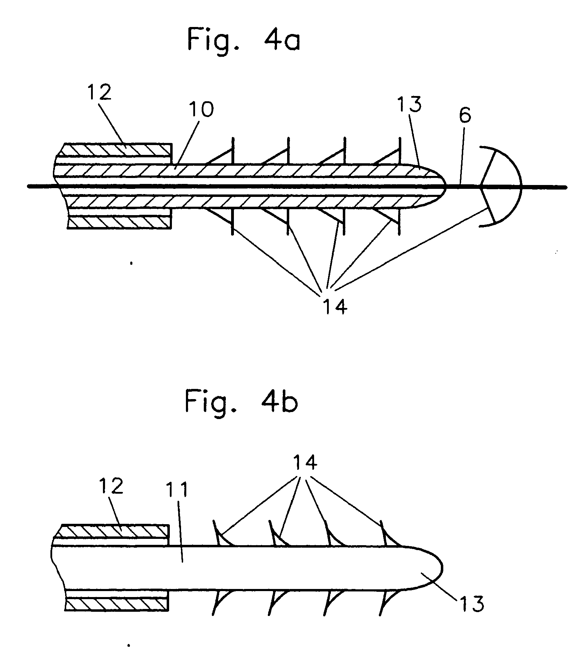

Neben den zuvor genannten Zangen als Applikationsvorrichtung zum Greifen und Setzen einzelner Clips zeigen die Figuren 4a und b einfache Vorrichtungen zum Applizieren von Clips, wobei in einer magazinähnlichen Komponente mehrere Clips 14 aufnehmbar und nacheinander applizierbar sind.

Bei beide Vorrichtungen gemäß den Figuren 4a und b sind die Clips 14 aufgebogen auf einen stangenförmigen Magazinkörper aufgeschoben. Der Magazinkörper kann ein Rohr 10, dargestellt in Fig. 4a mit aufgeschobenen Clips der ersten Ausführungsform, oder ein Vollkörper, dargestellt in Fig. 4b mit aufgeschobenen Clips der zweiten Ausführungsform, sein. Die aufgeschobenen Clips 14 lassen sich mit einer Überwurfhülse 12 nacheinander und kontrolliert über das distale Ende 13 des Magazinkörpers schieben und applizieren. Dabei ist für ein zuverlässiges separates Herunterschieben eines jeden Einzelclips ein Mindestabstand zwischen den Clips sicherzustellen. Dies ist Beispiel durch jeweils eine Verdrehung der Clips auf dem Rohr, aber auch durch Einsetzen von Abstandshalter vorzugsweise aus resorbierbaren Material jeweils zwischen zwei Clips sicherzustellen. Die Abstandshalter sind entweder separate Lochscheiben zwischen den Clips aufgeschoben oder an einer Seite, vorzugsweise am Biegeelement 5 eines jeden Clips als Formteile aufgesetzt. Hierdurch wird zudem ein Übereinanderschieben der Clips auf dem Magazinkörper entgegengewirkt.

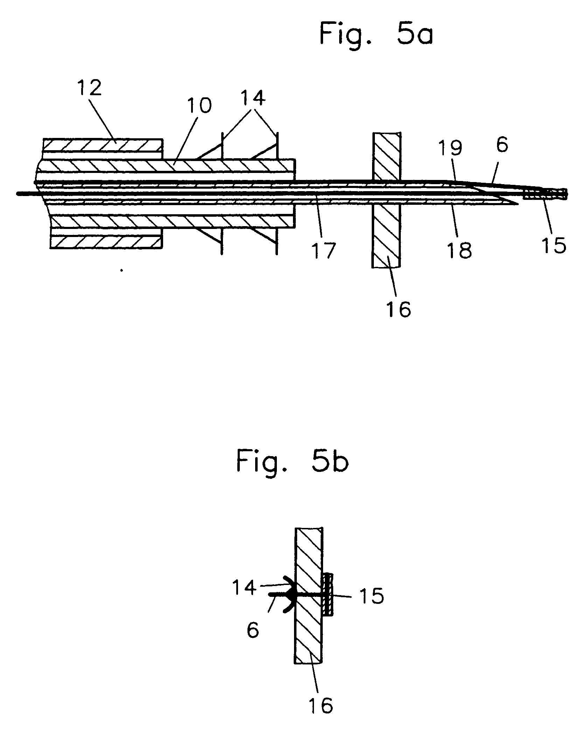

In Fig. 4 a ist zudem ein Faden 6 dargestellt, welcher sich durch das Rohr 10 zuführen lässt. Fig. 5 a zeigt diese Ausführungsform, ergänzt mit einer Zusatzeinrichtung zum Applizieren eines Clips 14 und eines Fadens 6 mit T-Anker 15. Einen mit dieser Zusatzeinrichtung applizierten Faden im Gewebe 16, gesichert durch den T-Anker und einem Clip ist in Fig. 5 b dargestellt. Der T-Anker besteht, wie auch in den Figuren 5 a und b dargestellt, aus Rohrstück mit einer mittigen Bohrung. In diese Bohrung ist das Ende des Fadens in Richtung eines Endes des Rohrstücks eingeschoben und ist in diesem Bereich durch ein lokales Zusammendrücken des Rohrstücks fixiert. Das Rohrstück besteht aus einem biokompatiblen, vorzugsweise resorbierbaren Material.

Zum Applizieren des Fadens 6 wird im Innern des Rohrs 10 eine Hohlnadel 18 mit einem schräg abgeschnittenen distalen Ende eingeführt. Diese ist bei dieser Ausführungsform am distalen Ende mit einem kurzen distal offenen Schlitz 19 versehen. Die Hohlnadel dient als Aufnahme des T-Ankers, wobei der Faden 6 vom T-Anker in der beschriebenen Ausführungsform durch den Schlitz hindurch in den Zwischenraum zwischen Hohlnadel und Rohr 10 geführt wird. Weist die Hohlnadel einen ausreichenden Innendurchmesser auf, ist der Faden auch in dieser zuleitbar, wobei der offene Schlitz ebenfalls nicht erforderlich wäre. Ferner befindet sich zum Applizieren des T-Stücks ein Führungsdraht 17 in der Hohlnadel, dessen distales Ende in das noch freie Rohrstück des T-Ankers eingeschoben ist und diesen in der gewünschten Position aus der Hohlnadel herausdrückt. Fig. 5 a zeigt die durch ein Gewebe 16 gestochene Hohlnadel 18, bei der das T-Stück bereits durch den Führungsdraht distal herausgeschoben wurde. Im Anschluss daran werden Hohlnadel und Führungsdraht mit dem Faden 6in das Rohr 10 zurückgezogen, wobei sich der Faden spannt, der T-Anker sich dabei querstellt und sich im Gewebe 16 verankert. Auf den gespannten Faden wird dann ein Clip 14 aufgeschoben (vgl. Fig. 5 b).

Das Rohr 10 eignet sich zudem zum Durchschieben eines Endoskops, was eine exakte visuelle Auswahl der Applikationsfläche einerseits und eine direkte visuelle Kontrolle der applizierten Clips noch während des Eingriffs andererseits auch bei minimalinvasiven Eingriffen ermöglicht.

Die Vorrichtungen gemäß der Figuren 4 a, b und 5 a eignen sich jedoch nur zum Applizieren von medizinischen Clips. Eine spätere Entfernung der Clips erfolgt beispielsweise mit den im Zusammenhang mit den beiden Ausführungsformen beschriebenen Zangen.

### Bezugszeichenliste

- 1: Plättchen
- 2: H-förmiger Durchbruch, Struktur
- 3: Lasche
- 4: Stirnseite
- 5: Biegeelement
- 6: Faden
- 7: Knickstelle
- 8: Wundränder
- 9: Organ
- 10: Rohr
- 11: Vollkörper
- 12: Überwurfhülse
- 13: Distale Ende
- 14: Clip
- 15: T-Anker
- 16: Gewebe
- 17: Führungsdraht
- 18: Hohlnadel
- 19: Schlitz

## Patentansprüche

1. Medizinischer Clip, bestehend aus einem gekrümmten Plättchen (1) aus einem elastischen biokompatiblen Material, in dem ein Durchbruch (2) unter Bildung zweier Laschen (3) mit je einer Stirnseite (4) als Greifelemente eingebracht ist, wobei sich die beiden Stirnseiten gegenüberstehen, und die beiden Laschen von dem Plättchen abgebogen sind, und wobei die Bereiche (5) der Plättchen, die jeweils seitlich außerhalb der Laschen liegen und die beiden Laschen auf beiden Seiten miteinander Verbinden, Biegeelemente bilden welche eine elastische Verspannung auf die Greifelemente gegeneinander bewirken.

2. Medizinischer Clip nach Anspruch 1, wobei die beiden Laschen in Innenrichtung des gekrümmten Plättchens eingebogen sind.

3. Medizinischer Clip nach Anspruch 1, wobei die beiden Laschen in Bezug auf das gekrümmte Plättchen nach außen weggebogen sind.

4. Medizinischer Clip nach einem der Ansprüche 1 bis 3, wobei das elastische biokompatible Material ein Formgedächtnismaterial ist.

5. Medizinischer Clip nach einem der Ansprüche 1 bis 4, wobei die Stirnseiten der Laschen eine Verzahnung, einen rutschfesten Belag, eine raue Oberfläche oder eine Riffelung aufweist.

## Claims

1. Surgical clip, consisting of a curved plate (1) made from an elastic biocompatible material, in which an opening (2) has been introduced to form two tongues (3), each having a face (4) as gripping element, wherein the two faces lie opposite each other, and both tongues are curved away from the plate, and wherein the areas (5) of the plates which each lie laterally outside the tongues and connect the two tongues on both sides with each other form bending elements which effect an elastic pre-tensioning on the gripping elements with respect to each other.

2. Surgical clip according to claim 1, wherein both tongues are curved in towards the interior of the curved plate.

3. Surgical clip according to claim 1, wherein both tongues are curved outwards in relation to the curved plate.

4. Surgical clip according to one of claims 1 to 3, wherein the elastic biocompatible material is a shape memory material.

5. Surgical clip according to one of claims 1 to 4, wherein the faces of the tongues have an indented surface, a non-skid coating, a coarse surface or ribbing.

## Revendications

1. Pince chirurgicale composée d'une plaquette courbe (1) formée d'une matière élastique, biocompatible, munie d'un passage (2) formant deux pattes (3) ayant chacune une face frontale (4) comme élément de préhension,
- les deux faces frontales étant situées l'une en regard de l'autre, et les deux pattes étant recourbées à partir de la plaquette, et
- les zones (5) des plaquettes respectives latéralement au-delà des pattes, et relient les deux pattes sur les deux côtés, constituent des éléments flexibles,
- ces éléments exerçant une précontrainte élastique entre les éléments de préhension.

2. Pince chirurgicale selon la revendication 1,
**caractérisée en ce que**
les deux pattes sont recourbées dans la direction intérieure de la plaquette cintrée.

3. Pince chirurgicale selon la revendication 1,
**caractérisée en ce que**
les deux pattes sont recourbées vers l'extérieur en s'écartant par rapport à la plaquette cintrée.

4. Pince chirurgicale selon l'une des revendications 1 à 3,
**caractérisée en ce que**
la matière élastique biocompatible est une matière à mémoire de forme.

5. Pince chirurgicale selon l'une des revendications 1 à 4,
**caractérisée en ce que**
les faces frontales des pattes comportent une denture, un revêtement non glissant, une surface rugueuse ou un moletage.
